Europäisches Patentamt

European Patent Office

Office européen des brevets

(19)

(11) Publication number: **0 098 100**
**B1**

(12) **EUROPEAN PATENT SPECIFICATION**

(45) Date of publication of patent specification: **22.10.86**

(51) Int. Cl.⁴: **A 61 B 1/00**

(21) Application number: **83303614.8**

(22) Date of filing: **23.06.83**

(54) Flexible tube for an endoscope.

(30) Priority: **24.06.82 JP 107601/82**

(43) Date of publication of application:
**11.01.84 Bulletin 84/02**

(45) Publication of the grant of the patent:
**22.10.86 Bulletin 86/43**

(84) Designated Contracting States:
**AT BE CH DE FR GB IT LI LU NL SE**

(56) References cited:
**DE-A-2 006 406**
**FR-A-2 282 080**
**FR-A-2 285 564**
**US-A-3 498 286**
**US-A-3 691 001**
**US-A-3 998 216**

(73) Proprietor: **OLYMPUS OPTICAL CO., LTD.**
**43-2, 2-chome, Hatagaya Shibuya-ku**
**Tokyo 151 (JP)**

(72) Inventor: **Takagi, Takeji**
**3092-1, Aihara-machi**
**Machida-shi Tokyo 194-02 (JP)**

(74) Representative: **Jones, Colin et al**
**W.P. THOMPSON & CO. Coopers Building**
**Church Street**
**Liverpool L1 3AB (GB)**

EP 0 098 100 B1

Courier Press, Leamington Spa, England.

## Description

The present invention relates to an endoscope.

An endoscope is generally formed of a forward end portion having an objective lens, a control body having an eyepiece, a bendable portion and flexible tube connected between the forward end portion and the control body. The insert portion, i.e., the forward end portion, the bendable portion and the flexible tube are inserted in the cavity of a human body, and the bendable portion is bent and operated by the control body to perform desired observations and/or operations.

The flexible tube has as an innermost layer a tubular core formed by a helically wound metallic strip to prevent collapsing of the tube. A cylindrical braid envelopes the core to prevent the flexible tube from twisting, and a sheath formed by, e.g., a plastic tube, covers the braid. The sheath is heated to melt a portion thereof into the interstices of the cylindrical braid to secure the sheath to the braid. Alternatively, a suitable adhesive can be inserted between the sheath and the braid to secure them to each other. On both ends of the core and the braid, ring-like end pieces are integrally mounted by brazing.

A conventional cylindrical braid for the flexible tube of such an endoscope is formed by a network of metallic wires, When a sheath of plastics material is adhered to the braid by heating or adhesive, sufficient bonding strength between the metal and the plastics material is not easily obtained. If separation should occur between the sheath and the braid, buckling of the flexible tube ensues, leading to failure of the endoscope.

US—A—3,691,001 discloses that, to improve the bonding strength between a sheath and a braid which is fitted snugly about an optical fibre bundle, the braid may be formed by interwoven metallic fibres and non-metallic fibres of natural and/or synthetic fibres, e.g., silk, cotton or polyamide.

However, end pieces must be attached to both ends of a flexible tube for an endoscope, and if a braid made of interwoven metallic and non-metallic fibres were to be brazed to metallic end pieces, the non-metallic fibres forming a portion of the braid would tend to melt or oxidize and so interfere with the brazing between the end pieces and the braid. Consequently, the brazing operation would become difficult, a predetermined tensile strength of the flexible tube would not easily be obtained, and further, some of the described non-metallic fibres cannot withstand acid or alkali which forms a component of the usual brazing flux.

An object of the present invention is to overcome the above-mentioned disadvantage and to provide a flexible tube for an endoscope having strong bonding strength between the sheath and the cylindrical braid and also having strong securing strength between the braid and the end pieces.

According to the present invention, a flexible tube for an endoscope having end pieces secured thereto and having a tubular core made from helically wound metallic strip, a cylindrical braid member fitted over the core, and a plastics sheath secured to the outer surface of the braid member, in which the braid member is braided of metallic wires and a plurality of non-metallic fibres, is characterised in that said non-metallic fibres are heat resistant to more than 150°C and are chemicals-resistant and are made from acid-resistant and alkali-resistant material, and in that the metallic annular end pieces are soldered to the metallic wires and to the metallic core.

Thus, the sheath is secured very strongly to the non-metallic fibres of the braid, and the end pieces can be soldered to the metallic wires of the braid without affecting the non-metallic filaments or fibres by heat and soldering flux.

The invention is further described, by way of example, with reference to the accompanying drawings, in which:—

Figure 1 is a schematic side view of an endoscope,

Figure 2 is a partially broken enlarged side view of a portion of the flexible tube of the endoscope shown in Figure 1, and

Figures 3 and 4 are enlarged diagrammatic views of a portion of cylindrical braid of the flexible tube respectively and showing two embodiments of the present invention.

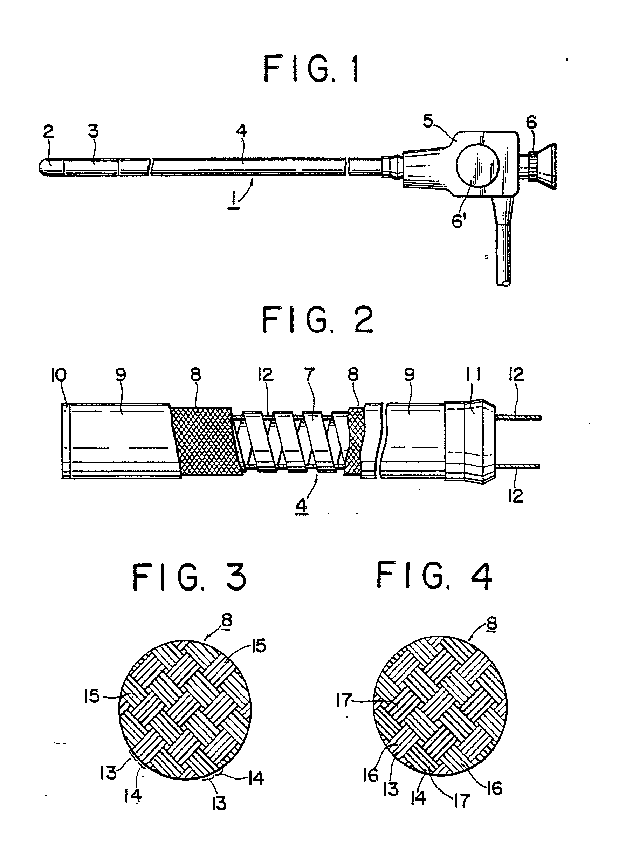

At first, a conventional endoscope is explained referring to Figures 1 and 2. As shown in Figure 1, an endoscope 1 has a forward end portion 2, a bendable portion 3, a flexible tube 4 and a control body 5 which has an eyepiece 6 and an operating knob 6' for controlling bending of the bendable portion 3. The forward end portion 2, the bendable portion 3 and the flexible tube 4 enclose a light guide (not shown) formed of an optical fibre bundle, for transmitting illuminating light to illuminate a desired region of a human body cavity, an image guide (not shown), formed of an optical fibre bundle for transmitting an image of the illuminated region, air and water feeders, and a forceps passage. The forward end of the forward end portion has outlet and inlet means for the light guide, the image guide, air and water outlets and a forceps outlet.

The flexible tube 4 is shown in Figure 2 and has a tubular core 7 which is formed by a helically wound metallic strip as an innermost layer to prevent the flexible tube 4 from collapsing. Covering the core 7 is a cylindrical braid 8 made of braided fibres to prevent the flexible tube 4 from twisting. On the whole outside surface of the braid 8, a sheath 9 made of plastics tube covers the braid 8. The sheath 9 is heated to melt a portion thereof into the interstices of the braid 8 to secure the sheath to the braid. Alternatively, suitable adhesive may be applied to the surface of the braid 8 and the sheath 9 is adhered to the braid 8. Before the braid 8 is covered by the sheath 9, a ring-like end piece 10 is integrally secured by brazing to one end of the core 7 and to the braid 8 and serves to connect the flexible tube to the bendable portion 3. Also a rear end piece 11 is secured by brazing to the other end of the core 7 and the braid 8 to connect the flexible tube to the control body 5. The brazing is performed by low melting point soldering metal. Two slender flexible sheaths 12 pass through the flexible tube and are secured to the front end piece 10 by soldering, the sheaths being each formed by a spirally wound strip. An operating wire (not shown) extends from the operating knob 6' of the control body 5 and through each slender sheath 12 to bend the bendable portion 3.

In the above described flexible tube, the cylindrical braid 8 is conventionally made of metallic wires e.g. stainless steel and brass which are braided together. In this case, when the outer sheath 9 which is made of plastics material is heated and secured to the braid 8 by melting a portion of plastics sheath into the mesh of the braid, the bonding strength between the sheath and the braid is relatively low and the bonding tends to separate.

To improve the bonding strength between a braid and an overlying sheath, the above-mentioned US—A—3,691,001 utilizes a braid which is made of interwoven metallic wires and non-metallic fibres, e.g. silk or cotton. In this case, the sheath 9 is bonded sufficiently strongly to the interwoven non-metallic fibres, by heating the sheath to melt a portion of the sheath into the interstices of the braid or by applying adhesive between the sheath and the braid. However, if the metallic end pieces 10 and 11 were to be brazed or soldered to the two ends of the braid 8, even at a relatively low temperature, the non-metallic fibres which constitute a portion of the braid 8 would tend to melt or oxidize under the applied heat and/or soldering flux. Consequently, the soldering operation would be disturbed. Further, as the non-metallic fibres would degenerate at both end portions of the braid, the bonding to the sheath would also deteriorate. Also, as the non-metallic fibres form a portion of the strength elements of the braid to resist tensile force, the tensile strength of the flexible tube would be decreased.

Figure 3 shows an enlarged view of a portion of a cylindrical braid, according to the present invention, for a flexible tube of an endoscope. In Figure 3, the cylindrical braid 8 is made of metallic wires 13 and heat-resistant, chemicals-resistant non-metallic mono-filaments, multi-filaments or fibres 14 which are interwoven to form the braid 8. The filaments or fibres are hereinafter referred to collectively as "fibres 14". In this embodiment, a plurality of metallic wires 13 and a plurality of heat-resistant, chemicals-resistant non-metallic fibres 14 are combined to form a wires and fibres group 15. Normally the group 15 is formed by two to ten strands, and in the embodiment shown in Figure 3, the group 15 is formed by three metallic wires 13 and two non-metallic fibres 14. A plurality of such wires and fibres group 15 is braided diagonally to form the cylindrical braid 8.

To braid such cylindrical braid 8 from the wires and fibres groups 15, each of which is formed by metallic wires 13 and non-metallic fibres 14, a plurality of bobbins each wound of the wires and fibres group 15 are prepared and the wires and fibres groups 15 are braided into the cylindrical braid 8 by suitable braiding machine.

Twist resistant material, e.g. stainless steel wires and brass wires are suitable as the metallic wires 13 of which the cylindrical braid 8 is formed.

For the heat-resistant, chemicals-resistant non-metallic fibres 14 to combine with the metallic wires 13, a suitable material must be chosen such that the fibres 14 do not disturb the soldering operation when the end pieces 10 and 11 are soldered, and such that the fibres 14 adhere strongly to the sheath 9. When the sheath 9 is heated to melt a portion of the sheath into the interstices of the braid 8, the melting temperature of the plastics material which form the sheath 9, e.g., polyurethane resin, polyester resin or polyvinyl chloride resin, is generally about 150° to 230°C. Thus, the non-metallic fibres 14 must be formed by material which will withstand a temperature of at least 150°C. Also, the braid 8 and the core 7 are soldered to the end pieces 10 and 11 by relatively low melting point soldering metal, i.e. one with a melting point of about 150°C or more, so that the fibres 14 must withstand the soldering temperature and also chemical action of soldering flux, i.e. acid or alkali.

Such heat-resistant and chemicals-resistant non-metallic materials which withstand melting temperatures of the plastics sheath and soldering metal and withstand acid and alkali and which can be formed into desired fibres 14, are organic and inorganic materials, such as polyimide, carbon, silicon carbide, boron nitride and glass. More than one material can be combined. In such materials, considering the affinity with the sheath 9 or adhesives and mechanical strength, a preferable material is polyimide.

In the embodiment shown in Figure 4, a plurality of metallic wires 13 forms a metallic wires group 16, and a plurality of non-metallic fibres 14 forms a non-metallic fibres group 17. Generally, fewer than ten wires 13 or fibres 14 for a respective group, and, in the embodiment shown in Figure 4, five wires 13 or five fibres 14 form each group. A plurality of wires groups 16 and fibres groups 17 is prepared and the groups are braided diagonally by a suitable braiding machine to form the interwoven cylindrical braid 8.

The mixture ratio of the metallic wires 13 and the heat-resistant, chemically stable non-metallic fibres 14 may be varied corresponding to outside diameter of the flexible tube.

To secure the sheath about the cylindrical braid 8, conventionally, the sheath is heated after the sheath 9 is placed over the braid 8 to melt a portion thereof into the interstices of the braid 8, or suitable adhesive is applied to the braid 8, and the sheath 9 is then placed over the braid 8. Plastics material may be applied to the braid 8 in a molten state and form the sheath after curing. In this case, affinity between the non-metallic fibres 14 and the sheath material forms a strong bond.

## Claims

1. A flexible tube for an endoscope adapted to be inserted in a body cavity, having end pieces (10, 11) secured thereto and having a tubular core (7) made of helically wound metallic strip, a cylindrical braid member (8) fitted over the core (7) and a plastics sheath (9) secured to the outer surface of the braid member (8), in which the braid member (8) is braided of a plurality of metallic wires (13) and a plurality of non-metallic fibres (14), characterised in that said non-metallic fibres (14) are heat-resistant to more than 150°C and are chemicals-resistant and are made from acid-resistant and alkali-resistant material, and in that the metallic annular end pieces (10, 11) are soldered to the metallic wires (13) and to the metallic core (7).

2. A flexible tube as claimed in claim 1, in which said non-metallic fibres (14) which form a portion of the cylindrical braid are made of one or more heat-resistant and chemicals-resistant materials including polyimide, carbon, silicon carbide, boron nitride or glass.

3. A flexible tube as claimed in claim 1 or 2, in which said sheath (9) is secured to the cylindrical braid (8) by melting a portion of the sheath material into the interstices of the braid.

4. A flexible tube as claimed in claim 1 or 2, in which said sheath (9) is secured to the cylindrical braid (8) by means of adhesive applied to the outer surface of the braid (8).

5. A flexible tube as claimed in claim 1 or 2, in which said sheath (9) is formed by applying molten plastics material to the outer surface of the braid (8).

## Patentansprüche

1. Biegsames Rohr für ein in einen Körperhohlraum einzuführendes Endoskop, mit daran befestigten Endstücken (10, 11) und einem rohrförmigen Kern (7), der aus einem schraubenförmig gewundenen Metallstreifen besteht, mit einem zylindrischen Flechtglied (8), das auf den Kern (7) gesetzt ist, und mit einer Kunststoffhülle (9), die an der Außenfläche des Flechtgliedes (8) befestigt ist, wobei dieses Flechtglied (8) aus einer Vielzahl von Metalldrähten (13) und einer Vielzahl von nichtmetallischen Fasern (14) geflochten ist, dadurch gekennzeichnet, daß die nichtmetallischen Fasern (14) gegenüber einer Temperatur von mehr als 150°C wärmebeständig und gegenüber einem chemischen Angriff widerstandsfähig sind und dabei aus einem säurefesten und alkalifesten Material hergestellt sind, und daß die metallischen, ringförmigen Endstücke (10, 11) an die Metalldrähte (13) und den Metallkern (7) gelötet sind.

2. Biegsames Rohr nach Anspruch 1, bei dem die nicht-metallischen Fasern (14), die einen Teil des zylindrischen Flechtwerks bilden, aus einem oder mehreren wärmebeständigen und chemisch beständigen Materialien besteht, einschließlich Polyimid, Kohlenstoff, Silicium, Carbid, Bornitrid oder Glas.

3. Biegsames Rohr nach Anspruch 1 oder 2, bei dem die Hülle (9) an dem zylindrischen Flechtwerk (8) durch Schmelzen eines Teils des Hüllenmaterials in Zwischenräume des Flechtwerks befestigt wird.

4. Biegsames Rohr nach Anspruch 1 oder 2, bei dem die Hülle (9) an dem zylindrischen Flechtwerk (8) mittels eines Klebstoffs befestigt ist, welcher auf die Außenfläche des Flechtwerks (8) aufgebracht ist.

5. Biegsames Rohr nach Anspruch 1 oder 2, dadurch gekennzeichnet, daß die Hülle (9) dadurch ausgebildet wird, daß geschmolzenes Kunststoffmaterial auf die Außenfläche des Flechtwerks (8) aufgebracht wird.

## Revendications

1. Tube souple pour un endoscope conçu pour être inséré dans une cavité de l'organisme, comprenant des embouts (10, 11) fixés à lui et possédant une âme tubulaire (7) faite de ruban métallique enroulé en hélice, une tresse cylindrique (8) disposée par-dessus l'âme (7) et une gaine en plastique (9) attachée à la surface extérieure de la tresse (8), dans lequel la tresse (8) est formée par tressage de fils métalliques (13) et de fibres non métalliques (14), caractérisé en ce que les fibres non métalliques (14) résistent à une température au-delà de 150°C et aux produits chimiques et sont faites de matériau résistant aux acides et aux alcalis, et que les embouts (10, 11), formés par des pièces métalliques annulaires, sont soudés aux fils métalliques (13) et à l'âme métallique (7).

2. Tube selon la revendication 1, dans lequel les fibres non métalliques (14), faisant partie de la tresse métallique, sont faites d'un ou de plusieurs matériaux qui résistent à la chaleur et aux produits chimiques, comprenant polyimide, carbone, carbure de silicium, nitrure de bore ou verre.

3. Tube selon la revendication 1 ou 2, dans lequel la gaine (9) est attachée à la tresse cylindrique (8) par fusion et pénétration d'une partie du matériau de la gaine dans les interstices de la tresse.

4. Tube selon la revendication 1 ou 2, dans lequel la gaine (9) est attachée à la tresse cylindrique (8) par de l'adhésif appliqué sur la surface extérieure de la tresse (8).

5. Tube selon la revendication 1 ou 2, dans lequel la gaine (9) est formée par application de matière plastique à l'état fondu sur la surface extérieure de la tresse (8).

# FIG. 1

2  3  4  5  6

1  6'

# FIG. 2

10  9  8  12  7  8  9  11  12

4  12  12

# FIG. 3

8  15

15

13  14  14  13

# FIG. 4

8

17  16

16  13  14  17